# EUROPEAN PATENT APPLICATION

(11) **EP 1 903 031 A2**
(43) Date of publication of application: **26.03.2008**
(21) Application number: 08150066.2
(22) Date of filing: 04.10.2005
(51) Int. Cl.: C07C 381/00

(54) **Synthesis of Pentafluorosulfanylnaphthalene**

(62) Divisional of application: 05021629.0
(71) Applicant: Air Products and Chemicals, Inc., Allentown, PA 18195-1501 (US)
(72) Inventor: Lal, Gauri Sankar, Whitehall, PA 18052 (US); Minnich, Kristen Elaine, Allentown, PA 18104 (US)
(74) Representative: Schwabe - Sandmair - Marx

(57) **Abstract**

This invention relates to 2-pentafluarasulfanylnapthalene, substituted derivatives thereof and to a process for producing naphthalene carrying an SF₅ group. A 3-step process is employed wherein 1,4-dihydronaphthalene is reacted with pentafluorosulfanyl halide. Next the resulting 3-halo-2-pentafluorosulfanylnaphthalene or derivative is converted to 2-pentafluorosulfanyl-1,4-dihydronaphthalene or derivative by treatment with a base and subsequently transformed into pentafluorosultanylnaphthalene by removing hydrogen atoms.

## Description

### BACKGROUND OF THE INVENTION

Fluorine incorporation in organic compounds is known to have a profound influence on the physical and chemical properties such as biological activity and electronegativity. A trifluoromethyl group has been employed as an effective means for introducing fluorine into such compounds. An alternative to trifluoromethyl incorporation has been the addition of sulfur pentafluoride. The high electronegativity value of the SF₅ group, 3.62 on the Pauling scale, and greater electron withdrawing ability suggest that it affords an attractive alternative to the trifluoromethyl group ("CF₃") found in many commercial products such as dye-stuffs, anesthetics, fluoropolymers, electronic components, and chemotherapeutic agents.

Pentafluorosulfanyl derivatives of aromatic compounds have been produced but the study of these compounds has been limited because of the lack of good synthetic routes. SF₅ addition to fused aromatic compounds is unknown.

Representative articles and patents illustrating addition of a sulfur pentafluoride group to organic compounds are as follows:

US 6,479,645 discloses the silyl sulfurpentafluoride and substituted ethyne compounds useful as a precursor for a variety of organic compounds. Substituted silyl acetylenic compounds are reacted with an SF₅ halide under condition to form an vinyl pentafluorosulfuanyl intermediate followed by addition of base. SF₅Br addition to trimethylsilylacetylene resulted in the product, pentafluorosulfuranyl-2-trimethyl silylethyne.

US 2003/0216476 A1 discloses the preparation of pentafluorosulfanylbenzoylguanidines. These compounds were found to be suitable as antiarrythmic agents and for treatment of angina pectoris.

JP 2004/059452A, discloses the preparation of N-ribo-benzimidazole bearing an SF₅ group. This compound was found to be an effective anti-viral and anti-cancer agent.

Kirsch, et. al in Agnew. Chem. Int. Ed. 1999, 38, 13, 1989 reported in the article, Liquid Crystals Based Upon Hypervalent Sulfur Fluorides: Pentafluorosulfuranyl as Polar Terminal Group*,* disclose the application of aryl sulfurpentafluorides as liquid crystals and demonstrated an improved electro-optical property of the -SF₅ group over the -CF₃ group. Pentafluorosulfuranylbenzene derivatives were produced by direct fluorination of deactivated aromatic disulfides.

Sheppard and co-workers, J. Am. Chem. Soc. 1962, 84, 3064 disclose in an article *Arylsulfur Pentafluorides,* the reaction of aryl disulfides with silver difluoride to obtain phenyl sulfurpentafluoride and nitrophenyl sulfurpentafluoride in -30% yield.

Bowden et al, Tetrahedron, 2000, 56, 3399, in an article A New Method for the Synthesis of Aromatic Sulfurpentafluorides and Studies of the Stability of the Sulfurpentafluoride Group in common Synthetic Transformations*,* disclose the reaction of aryl disulfides with F₂ in the presence of CH₃CN to produce nitrophenylsulfurpentafluorides. The nitrophenylsulfurpentafluorides then were converted to aminophenyl sulfurpentafluorides, biphenylsulfurpentafluoride, acetamidophenyl sulfurpentafluoride, and the like.

Hoover et al, J. Am. Chem. Soc. 1964, 3567 in an article entitled, Synthesis And Chemistry Of Ethynylsulfur Pentafluoride disclosed a procedure to phenyl sulfurpentafluoride and dimethylphenyl sulfurpentafluoride involving a Diels-Alder cycloaddition reaction of butadiene and 2,3-dimethylbutadiene respectively with ethynyl sulfurpentafluoride.

WO 011422A1 discloses the preparation of pentafluarosulfanylbenzene via the reaction of SF₅Cl with 4,5-dichloro-9-cyclohexene and pentasulfanylalkenes by reacting SF₅Cl with an alkene.

### BRIEF SUMMARY OF THE INVENTION

This invention relates to pentafluorosulfanyl derivatives of fused aromatic compounds and particularly to the compounds, 2-pentafluorosulfanylnapthalene and substituted derivatives thereof, and to a process for producing naphthalene carrying an SF₅ group. A 3-step process is preferred wherein 1,4-dihydronaphthalene is reacted with SF₅Br in the presence of B(Et)₃. Next the resulting 3-bromo-2-pentafluorosulfanylnaphthalene is converted to 2-pentafluorosulfanyl-1,4-dihydronaphthalene by treatment with a base and subsequently transformed into pentafluorsulfanylnaphthalene by oxidation with 2,3-dichloro-5,6-dicyano-benzoquinone (DDQ.)

Significant advantages are achieved by the process and the synthesis of the fused aromatic compounds incorporating an SF₅ group, e.g., 2-pentafluorosulfanylnapthalene and its derivatives, and they include:
an ability to incorporate an SF₅ group onto a fused aromatic compound, e.g., naphthalene or derivative and thus provide for unique properties in a fused aromatic;
an ability to generate fluorinated organics having suitability for producing liquid crystal components;
an ability to produce materials having electro-optical properties;
an ability to produce fused aromatic compounds incorporating an SF₅ group via a route which may lead to viable medicinal and agrochemical products; and,
an ability to use a 3 step process for the production of fused aromatic compounds having an SF₅ group.

### DETAILED DESCRIPTION OF THE INVENTION

As pointed out in the background, SF₅ incorporation into aromatic compounds has been limited because of a want of acceptable synthetic methods. SF₅ addition to fused aromatic compounds had not been an option for the organic chemist as SF₅ does not add directly. Routes employing direct fluorination of aromatic disulfides have not afforded a method for producing pentafluorosulfanyl derivatives of fused aromatic compounds either.

A process has been developed not only where SF₅ is incorporated into a fused aromatic, e.g., naphthalene but also where the bonding in the aromatic ring is conjugated in relationship to the SF₅ group. Thus, the compound exhibits unique electronic activity not only because of the SF₅ incorporation but also because of the conjugated bond structure.

In the first step of the process, 1,4-dihydronaphthalene is contacted with an SF₅ halide such as SF₅Br or SF₅Cl under conditions for effecting addition of the SF₅ group in the 2-position. This is necessary in the reaction scheme in order to achieve proper bond placement in subsequent process steps. The reaction is preferably carried out in the presence of triethylborane B(ET)₃ acting as a catalyst. Other catalysts may be employed and these include free radical generators, benzoyl peroxide and azo compounds and ultra violet light.

In the second step, assuming SF₅Br was the reactant in the first step, bromine is removed from the 3-bromo-2-pentafluorosulfanylnaphthalene produced in the first step. Dehydrobromination is effected by treatment of the brominated compound with a base under conditions for removing bromine. The base reactant suited for dehalogenation, e.g., dehydrobromination, may include alkali or alkaline earth metal hydroxides, alkoxides, amides, amines, metal alkyl derivatives. However, because the process requires removal of halide from the 3-position, the use of SF₅Br is preferred as a reactant in the first step because of ease of bromine removal. Should SF₅Cl have been used as the reactant, dehydrochlorination of the compound, 3-chloro-2-pentafluorosulfanylnaphthalene, would have been required and removal of chlorine is difficult in this kind of reaction.

In the third step, the 2-pentafluorosulfanyl-1,4-dihydronaphthalene or derivative formed in step 2 is oxidized, i.e, 2 hydrogen atoms are removed and the fused product, 2-pentafluorosulfanylnapthalene or derivative, formed. Oxidation or dehydrogenation of 2-pentafluorosulfanyl-1,4-dihydronaphthalone can be achieved by reaction with 2,3-dichloro-5,6-dicyano-benzoquinone (DDQ.) On oxidation with DDQ, conjugated bonding is achieved in the aromatic ring in relation to the SF₅ group. As such, the conjugated bonding, coupled with the SF₅ functionality provides for desired electronic characteristics. Other reagents which can effect dehydrogenation include benzoquinone and other oxidants such as O₂ in the presence of activated carbon, nitric acid, nitrites of alkali metals, salts of Ce, Cu, Bi, Mn, and Zr. Metals such as Pt, Pd should also be suitable.

The process steps for producing pentafluorosulfanyl naphthalenes and derivatives wherein the SF₅ group is in the 2-position are outlined below. 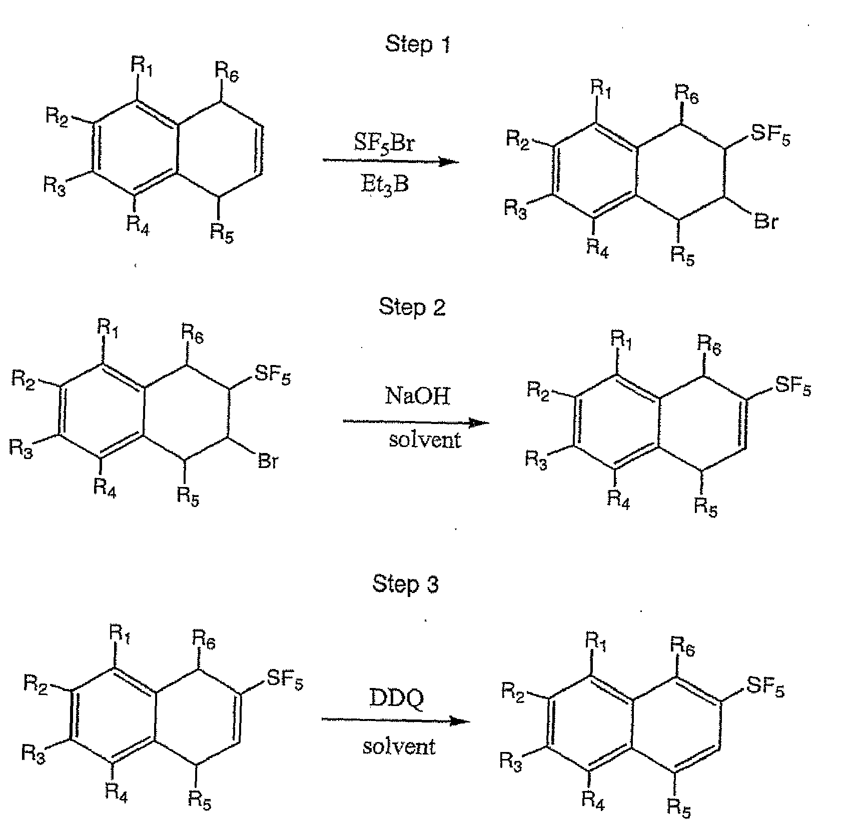 wherein R₁₋₄ are H, C₁₋₆ alkyl, substituted C₁₋₆ alkyl, aryl or substituted aryl; O, S, and N hetero compounds, -COOR₇ where R₇ is C₁₋₆ alky, -COR₈ where R₈ is C₁₋₆ alkyl or halogen, i.e., F, Cl. Br, and I; boron esters, (R₉)₃B where R₉ is C₁₋₆ alkyl, and R₅ and R₆ are H. Typically, only one or two substituents of R₁₋₄ are other than hydrogen may be appended to the ring. For example, one or two of R₁ to R₄ may be C₁ alkyl. In cases where substitution is desired, substitution on the R₃ is position is preferred, such as F, Cl, Br or I.

Substituted derivatives may be formed by forming a substituted 1,4-dihydronapthalene prior to contact and reaction with the SF₅ halide. Alternatively, substituents may be incorporated subsequent to formation of the desired compound. A problem with the second approach is that reaction may take place in the 7, 3, or 4 position of the fused aromatic

Representative named pentafluorosulfanylnapthalene derivatives are represented by the formula: 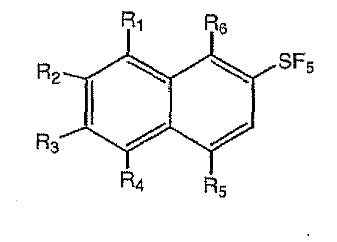 are as follows: 6-bromo-2--pentafluorosulfanylnaphthalene, 6-chloro-2-pentafluorosulfanylnaphthalene, 6-fluora-2-pentafluorosulfanylnaphthalene, 6-iodo-2-pentafluorosulfanylnaphthalene, 6-methyl-2-pentafluorosulfanylnaphthalene, 6-propyl-2-pentafluarosulfanylnaphthalene, 6-pentyl-2-pentafluorosulfanylnaphthalene, 6-cyclohexyl-2-pentafluorosulfanylnaphthalene, 6-(4-propylcyclohexyl)-2-pentafluorasulfanylnaphthalene, 6-carboethoxy-2-pentafluorosulfanylnaphthalene, 6-trimethylsilyl-2-pentafluorosulfanylnaphthalene, 6-trimethoxyborane-2- pentafluorosulfanylnaphthalene, and 8-trimethylborane-2-pentafluarosulfanylnaphthalene.

In the above series of reactions solvents may be used as necessary and these include hydrocarbons, fluorocarbons, nitriles, ethers, halocarbons, and other solvents, which will not react with reactants such as the base. Reaction temperatures typically range from -78 °C to the boiling point of solvent. Reaction products may be purified by standard methods including distillation and chromatography.

The following examples are provided to illustrate various embodiments and comparisons and are not intended to restrict the scope of the invention.

### Example 1

### Preparation of Step1

### Preparation of 3-bromo-2-pentafluorosulfanylnaphthalene

1,4 Dihydronaphthalene (2 g, 15.8 mmol), pentane (100 mL), potassium fluoride (0.3 g) and triethylborane (1.6 mL, 1M in hexanes) were charged to 300 cc ss Parr reactor. The solution was cooled and degassed. SF₅Br (18 mmol) was condensed into the reactor at -50 °C. The reaction was stirred for 1 hour then the cooling bath was removed and stirring was continued one hour. The solution was poured into cold sodium bicarbonate, phases were separated and pentane layer dried over magnesium sulfate.

### Step 2

### Preparation of the product pentafluorsulfanyl-1,4-dihydronaphthalene

Powdered sodium hydroxide (5 equivalents) was added to the pentane solution and stirred at room temperature. The reaction was monitored by GC. When the reaction was complete, base was removed by filtration and pentane was removed by rotary evaporation.

### Step 3

### Preparation of Pentafluorosulfanylnaphthalene

Toluene and dichlorodicyanobenzoquinone (2 equivalents) were added to the product of step 2 and heated to 65 °C. The reaction was monitored by GC. When the reaction was complete pentane was added. Resulting solids were removed by filtration. Color was removed by filtering through silica. The crude product was isolated by removing the solvent using rotary evaporation.

The product was purified by sublimation. Residual solvent and naphthalene byproduct were removed under vacuum at room temperature. The product sublimed at 35 °C/220 mTorr and was identified by mass spectrometry,¹H and ¹⁹F NMR.

The invention further relates to the following numbered embodiments:
1. A compound represented by the formula: 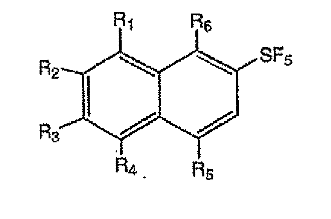 wherein R₁₋₄, are H, C₁₋₆ alkyl, substituted C₁₋₆ alkyl, aryl or substituted aryl; O, S, and N hetero, -COOR₇ where R₇ is alkyl, -COR₈ where R₈ is C₁₋₆ alkyl; F, Cl, Br, I; boron C₁₋₆ alkyl ester, (R₉)₃B where R₉ is C₁₋₆ alkyl, and R₅ and R₆ are H.
2. The compound of embodiment 1 wherein R₁₋₄, is H.
3. The compound of embodiment 1 wherein R₃ is C₁₋₆ alkyl and R₁, R₂, and R₄ are H.
4. The compound of embodiment 1 wherein R₃ is -COOR₇ where R₇ is C₁₋₆ alkyl and R₁, R₂, and R₄ are H.
5. The compound of embodiment 1 wherein R₃ is -COR₈ where R₈ is C₁₋₆ alkyl and R₁, R₂, and R₄ are H.
6. The compound of embodiment 1 wherein R₃ is a boron C₁₋₆ alkyl ester and R₁ R₂, and R₄ are H.
7. The compound of embodiment 1 wherein R₃ is, (R₉)₃B where R₉ is C₁₋₆ alkyl and R₁, R₂ and R₄ are H.
8. The compound of embodiment 1 wherein R₃ is halogen and R₁, R₂, and R₄ are H.
9. The compound of embodiment 8 wherein R₃ is F, Cl, Br, or 1.
10. A compound selected from the group consisting of 6-bromo-2-pentafluorosulfanylnaphthalene, 6-chloro-2-pentafluorosulfanylnaphthalene, 6-fluoro-2-pentafluorosulfanylnaphthalene, 6-iodo-2-pentafluorosulfanylnaphthalene, 6-methyl-2-pentafluorosulfanylnaphthalene, 6-propyl-2-pentafluorosulfanylnaphthalene, 6-pentyl-2-pentafluorosulfanylnaphthalene, 6-eyclahexyl-2-pentafluarosulfanylnaphthalene, 6-(4-propylcyclohexyl)-2-pentafluorosulfanylnaphthalene, 6-carboethoxy-2-pentafluorosulfanylnaphthalene, 6-trimethylsilyl-2-pentafluorosulfanylnaphthalene, 6-trimethoxyborane-2-pentafluorosulfanylnaphthalene, and 6-trimethylborane-2-pentafluorosulfanylnaphthalene.
11. A process for producing a compound of the formula A: 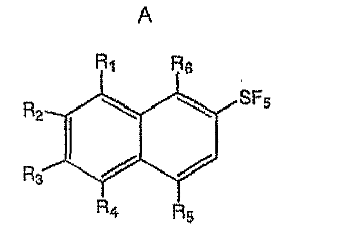 wherein R₁₋₄ are H, C₁₋₆ alkyl, substituted C₁₋₆ alkyl, aryl or substituted aryl; O, S, and N hetero, -COOR₇ where R₇ is C₁₋₆ alkyl, -COR₈ where R₈ is C₁₋₆ alkyl; F, Cl, Br, and I; boron C₁₋₆ esters, (R₉)₃B where R₉ is C₁₋₆ alkyl, and R₅ and R₆ are H which comprises the steps:
   (a) contacting a reactant of the formula B: 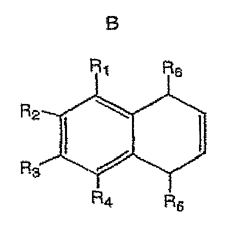 wherein R₁₋₄ are H, C₁₋₆ alkyl, substituted C₁₋₆ alkyl, aryl or substituted aryl; O, S, and N hetero, -COOR₇ where R₇ is C₁₋₆ alkyl, -COR₈ where R₈ is C₁₋₆ alkyl; F, Cl, Br, and I; boron C₁₋₆ esters, (R₉)₃B where R₉ is C₁₋₆ alkyl, and R₅ and R₆ are H with an SF₅ bromide under conditions for forming compound C: 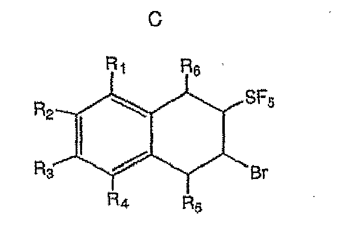 wherein R₁₋₄ are H, C₁₋₆ alkyl, substituted C₁₋₆ alkyl, aryl or substituted aryl; O, S, and N hetero, -COOR₇ where R₇ is C₁₋₆ alkyl, -COR₈ where R₈ is C₁₋₆ alkyl; F, Cl, Br, and I; boron C₁₋₆ esters, (R₉)₃B where R₉ is C₁₋₆ alkyl, and R₅ and R₆ are H;
   (b) dehydrobrominating compound C under conditions for forming compound D having the formula: 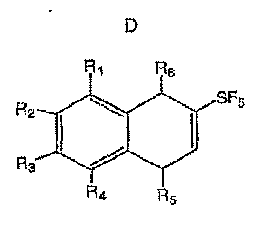 and then;
   (c) removing hydrogen from compound D under conditions for producing compound A.
12. The process of embodiment 11 wherein at least one of R₁, R₂ and R₄ are H.
13. The process of embodiment 11 wherein R₃ is C₁₋₆ alkyl.
14. A process for producing 2-pentafluorosulfanylnaphthalene which comprises the steps.
   (a) contacting 1,4-dihydronaphthalene with SF₅Br in the presence of a catalyst to form 3-bromo-2-pentafluorasulfanylnaphthalene;
   (b) removing bromine from the compound 3-bromo-2-pentafluorosulfanylnaphthalene formed in step (a) thus forming pentafluorosulfanyl-1,4-dihydronaphthalene; and,
   (c) removing two hydrogen atoms from the thus formed pentafluorosulfanyl-1,4-dihydronaphthalene in step (b) to produce 2-pentafluorosulfanylnaphthalene.
15. The process of embodiment 14 wherein the catalyst is triethylborane.
16. The process of embodiment 14 wherein bromine is removed by treatment with a base.
17. The process of embodiment 16 wherein the base is NaOH.
18. The process of embodiment 14 wherein two hydrogen atoms are removed in step (c) by reaction with dichlorodicyanobenzoquinone

## Claims

1. A compound represented by the formula: 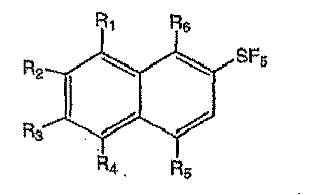 wherein R₁₋₄ are H, C₁₋₆ alkyl, substituted C₁₋₆ alkyl, aryl or substituted aryl; O, S, and N hetero, -COOR₇ where R₇ is C₁₋₆ alkyl, -COR₈ where R₈ is C₁₋₆ alkyl; F, Cl, Br, I; boron C₁₋₆ alkyl ester, (R₉)₃B where R₉ is C₁₋₆ alkyl, and R₅ and R₆ are H.

2. The compound of Claim 1 wherein R₁₋₄ is H.

3. The compound of Claim 1 wherein R₃ is C₁₋₆ alkyl and R_{1,} R₂, and R₄ are H.

4. The compound of Claim 1 wherein R₃ is -COOR₇ where R₇ is C₁₋₆ alkyl and R₁, R₂, and R₄ are H.

5. The compound of Claim 1 wherein R₃ is -COR₈ where R₈ is C₁₋₆ alkyl and R₁, R₂, and R₄ are H.

6. The compound of Claim 1 wherein R₃ is a boron C₁₋₆ alkyl ester and R₁, R₂, and R₄ are H.

7. The compound of Claim 1 wherein R₃ is, (R₉)₃B where R₉ is C₁₋₆ alkyl and R₁, R₂, and R₄ are H.

8. The compound of Claim 1 wherein R₃ is halogen and R₁, R₂, and R₄ are H.

9. The compound of Claim 8 wherein R₃ is F, Cl, Br, or 1.

10. A compound selected from the group consisting of 6-bromo-2-pentafluorosulfanylnaphthalene, 6-chloro- 2-pentafluorosulfanylnaphthalene, 6-fluoro-2-pentafluorosulfanylnaphthalene, 6-iodo-2-pentafluorosulfanylnaphthalene, 6-methyl-2-pentafluorosulfanylnaphthalene, 6-propy 1 -2-pentafluorosulfanylnaphthalene, 6-penty 1-2-pentafluorosulfanylnaphthalene, 6-cyclohexyl -2-pentafluorosulfanylnaphthalene, 6-(4-propylcyclohexyl)-2-pentafluorosulfanyinaphthalene, 6-carboethoxy-2-pentafluorosulfanylnaphthalene, 6-trimethyisilyl-2-pentafluorosulfanylnaphthalene, 6-trimethoxyborane-2-pentafluorosulfanylnaphthalene, and 6-trimethylborane-2-pentafluorosulfanylnaphthalene.
